(19) Europäisches Patentamt
European Patent Office
Office européen des brevets

(11) Veröffentlichungsnummer: **0 503 437 A1**

(12) # EUROPÄISCHE PATENTANMELDUNG

(21) Anmeldenummer: **92103589.5**

(51) Int. Cl.5: **C07D 249/14**, A01N 47/38

(22) Anmeldetag: **02.03.92**

(30) Priorität: **14.03.91 DE 4108187**

(43) Veröffentlichungstag der Anmeldung:
**16.09.92 Patentblatt 92/38**

(84) Benannte Vertragsstaaten:
**BE CH DE ES FR GB IT LI NL**

(71) Anmelder: **BAYER AG**

**W-5090 Leverkusen 1 Bayerwerk(DE)**

(72) Erfinder: **Findeisen, Kurt, Prof.Dr.**
**Dünfelder Strasse 28**
**W-5090 Leverkusen 1(DE)**
Erfinder: **Kuhnt, Dietmar Dr.**
**Körnerstrasse 5**
**W-5090 Leverkusen 1(DE)**
Erfinder: **Müller, Klaus-Helmut Dr.**
**Bockhackstrasse 55**
**W-4000 Düsseldorf 13(DE)**
Erfinder: **König, Klaus Dr.**
**Zum Hahnenberg 40**
**W-5068 Odenthal(DE)**
Erfinder: **Lürssen, Klaus Dr.**
**August-Kierspel-Strasse 145**
**W-5060 Bergisch Gladbach 2(DE)**
Erfinder: **Santel, Hans-Joachim Dr.**
**Grünstrasse 9a**
**W-5090 Leverkusen 1(DE)**
Erfinder: **Schmidt, Robert R. Dr.**
**Im Waldwinkel 110**
**W-5060 Bergisch Gladbach 2(DE)**

(54) **Substituierte Triazolinone.**

(57) Die Erfindung betrifft neue substituierte Triazolinone der Formel (I)

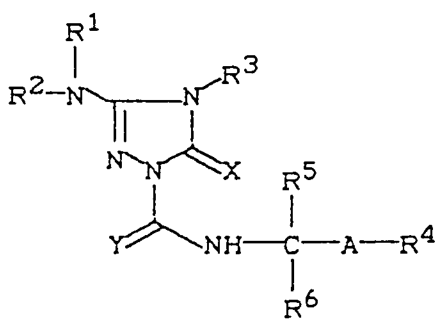

(I)

in welcher
A für einen Rest der Formel
$-CH_2-CH_2-$; $-CH_2-CH_2-CH_2-$;

EP 0 503 437 A1

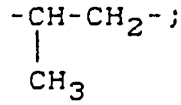 ; 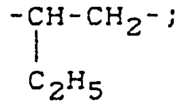 ; 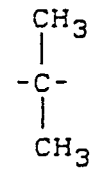

oder 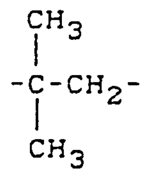

steht,

X und Y jeweils für O oder S stehen und die Reste $R^1$-$R^6$ die in der Beschreibung genannten Bedeutungen haben,

Verfahren zu ihrer Herstellung und ihre Verwendung als Herbizide.

Die Erfindung betrifft neue substituierte Triazolinone, Verfahren zu ihrer Herstellung und ihre Verwendung als Herbizide.

Es ist bekannt, daß bestimmte substituierte Triazolinone wie beispielsweise die Verbindung 1-(4-Phenyl-2-butylaminocarbonyl)-3-dimethylamino-4-methyl-1,2,4-triazolin-5-on herbizide Eigenschaften besitzen (vergleiche z. B. DE-OS 37 09 574).

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden neue substituierte Triazolinone der allgemeinen Formel (I) gefunden,

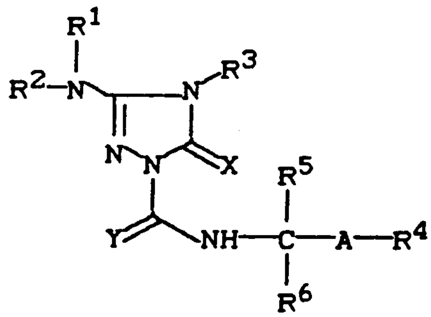

(I)

in welcher

A für einen Rest der Formel
$-CH_2-CH_2-$; $-CH_2-CH_2-CH_2-$;

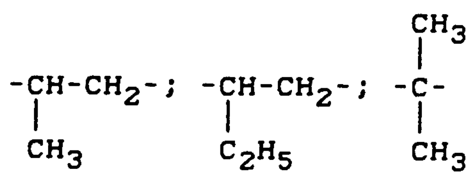

oder 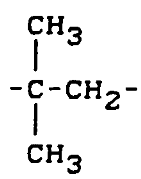

steht,

X für Sauerstoff oder Schwefel steht,

Y für Sauerstoff oder Schwefel steht,

$R^1$ für Wasserstoff oder Alkyl steht,

$R^2$ für Alkyl steht,

$R^3$ für Alkyl oder Alkenyl steht,

$R^4$ für jeweils gegebenenfalls substituiertes Cycloalkyl oder Aryl steht,

$R^5$ für Alkyl steht oder auch für Wasserstoff steht, für den Fall, daß nicht gleichzeitig A für $-CH_2-CH_2-$ steht und $R^4$ für unsubstituiertes Phenyl steht und

$R^6$ für Alkyl oder Cyano steht oder auch für Wasserstoff steht, für den Fall, daß A gleichzeitig für einen verzweigten Alkandiylrest der Formel

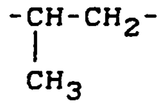; 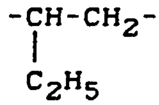; 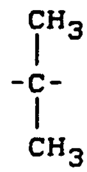 oder 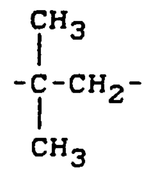

steht,

oder

$R^5$ und $R^6$ gemeinsam für einen zweifach verknüpften Alkandiylrest stehen.

Weiterhin wurde gefunden, daß man die neuen substituierten Triazolinone der allgemeinen Formel (I),

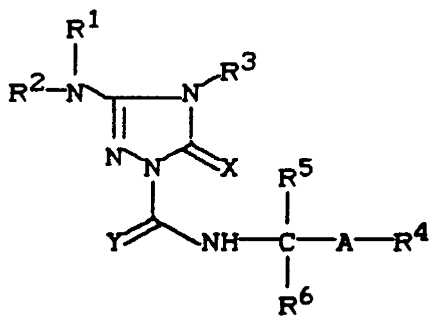

(I)

in welcher

A für einen Rest der Formel

$-CH_2-CH_2-$; $-CH_2-CH_2-CH_2-$;

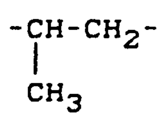; 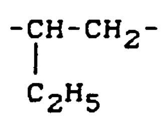; 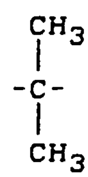

oder 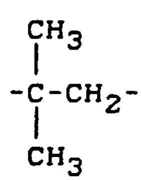

steht,

X für Sauerstoff oder Schwefel steht,

Y für Sauerstoff oder Schwefel steht,

$R^1$ für Wasserstoff oder Alkyl steht,

$R^2$ für Alkyl steht,

$R^3$ für Alkyl oder Alkenyl steht,

$R^4$ für jeweils gegebenenfalls substituiertes Cycloalkyl oder Aryl steht,

$R^5$ für Alkyl steht
oder auch für Wasserstoff steht, für den Fall, daß nicht gleichzeitig A für $-CH_2-CH_2-$ steht und $R^4$ für unsubstituiertes Phenyl steht und

$R^6$ für Alkyl oder Cyano steht
oder auch für Wasserstoff steht, für den Fall, daß A gleichzeitig für einen verzweigten Alkandiylrest der Formel

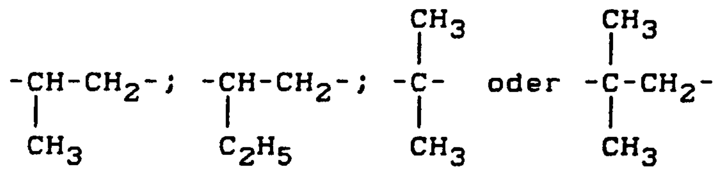

oder

steht,
oder

$R^5$ und $R^6$ gemeinsam für einen zweifach verknüpften Alkandiylrest stehen,

erhält, wenn man

a) 1-Chlor-(thio)-carbonyltriazolinone der Formel (II),

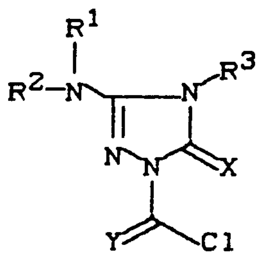

(II)

in welcher
$R^1$, $R^2$, $R^3$, X und Y die oben angegebene Bedeutung haben,
mit Aminen der Formel (III),

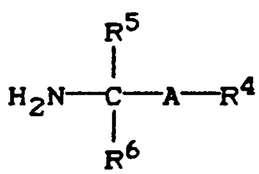

(III)

in welcher
$R^4$, $R^5$, $R^6$ und A die oben angegebene Bedeutung haben,
gegebenenfalls in Gegenwart eines Verdünnungsmittels und gegebenenfalls in Gegenwart eines Reaktionshilfsmittels umsetzt,
oder wenn man
b) in 1-Stellung unsubstituierte Triazolinone der Formel (IV),

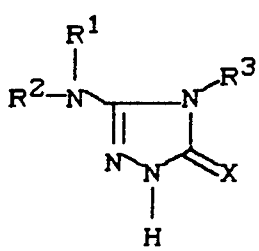

(IV)

in welcher
$R^1$, $R^2$, $R^3$ und X die oben angegebene Bedeutung haben,
mit Iso-(thio)-cyanaten der Formel (V),

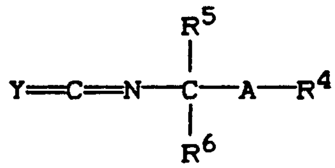 (V)

in welcher
A, Y, $R^4$, $R^5$ und $R^6$ die oben angegebene Bedeutung haben,
gegebenenfalls in Gegenwart eines Verdünnungsmittels und gegebenenfalls in Gegenwart eines Reaktionshilfsmittels umsetzt.

Schließlich wurde gefunden, daß die neuen substituierten Triazolinone der allgemeinen Formel (I) herbizide Eigenschaften besitzen.

Überraschenderweise zeigen die erfindungsgemäßen substituierten Triazolinone der Formel (I) eine erheblich bessere herbizide Wirksamkeit gegenüber Problemunkräutern und gleichzeitig eine vergleichbar gute Verträglichkeit gegenüber wichtigen Kulturpflanzen im Vergleich zu den aus dem Stand der Technik bekannten substituierten Triazolinonen, wie beispielsweise die Verbindung 1-(4-Phenyl-2-butylaminocarbonyl)-3-dimethylamino-4-methyl-1,2,4-triazolin-5-on, welche chemisch und wirkungsmäßig naheliegende Verbindungen sind.

Die erfindungsgemäßen substituierten Triazolinone sind durch die Formel (I) allgemein definiert. Bevorzugt sind Verbindungen der Formel (I), bei welchen

A für einen Rest der Formel
$-CH_2-CH_2-$; $-CH_2-CH_2-CH_2-$;

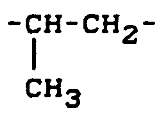 ; 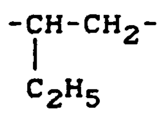 ; 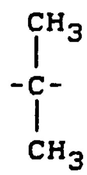

oder 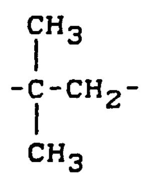

steht,

X für Sauerstoff oder Schwefel steht,

Y für Sauerstoff oder Schwefel steht,

$R^1$ für Wasserstoff oder für geradkettiges oder verzweigtes Alkyl mit 1 bis 6 Kohlenstoffatomen steht,

$R^2$ für geradkettiges oder verzweigtes Alkyl mit 1 bis 6 Kohlenstoffatomen steht,

$R^3$ für geradkettiges oder verzweigtes Alkyl mit 1 bis 6 Kohlenstoffatomen oder für geradkettiges oder verzweigtes Alkenyl mit 2 bis 6 Kohlenstoffatomen steht,

$R^4$ für gegebenenfalls einfach oder mehrfach, gleich oder verschieden substituiertes Cycloalkyl mit 3 bis 7 Kohlenstoffatomen steht, wobei als Substituenten infrage kommen: geradkettiges oder verzweigtes Alkyl mit 1 bis 4 Kohlenstoffatomen oder für jeweils gegebenenfalls einfach oder mehrfach, gleich oder verschieden substituiertes Phenyl oder Naphthyl steht, wobei als Substituenten infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen, geradkettiges oder verzweigtes Alkoxycarbonyl oder Alkoximinoalkyl mit jeweils 1 bis 4 Kohlenstoffatomen in den einzelnen Alkylteilen; Dialkylamino mit jeweils 1 bis 4 Kohlenstoffatomen in den einzelnen geradkettigen

oder verzweigten Alkylteilen, N-Alkanoylamino mit 1 bis 5 Kohlenstoffatomen im geradkettigen oder verzweigten Alkanoylteil, gegebenenfalls einfach oder mehrfach, gleich oder verschieden durch Halogen substituiertes, zweifach verknüpftes Dioxyalkylen mit 1 bis 3 Kohlenstoffatomen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl,

$R^5$ für geradkettiges oder verzweigtes Alkyl mit 1 bis 6 Kohlenstoffatomen steht oder auch für Wasserstoff steht, für den Fall, daß nicht gleichzeitig A für $-CH_2-CH_2-$ und $R^4$ für unsubstituiertes Phenyl steht und

$R^6$ für Cyano oder für geradkettiges oder verzweigtes Alkyl mit 1 bis 6 Kohlenstoffatomen steht oder auch für Wasserstoff steht, für den Fall, daß A gleichzeitig für einen verzweigten Alkandiylrest der Formel

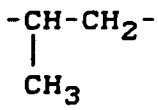 ; 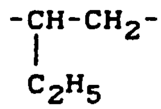 ; 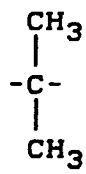 oder 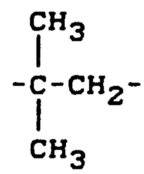

steht,

oder

$R^5$ und $R^6$ gemeinsam für einen zweifach verknüpften Alkandiylrest mit 2 bis 7 Kohlenstoffatomen stehen.

Besonders bevorzugt sind Verbindungen der Formel (I), bei welchen

A für einen Rest der Formel

$-CH_2-CH_2-$; $-CH_2-CH_2-CH_2-$;

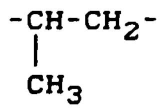 ; 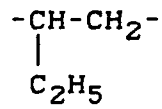 ; 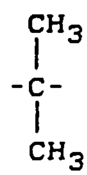

oder 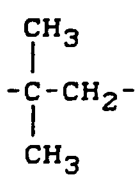

steht,

X für Sauerstoff oder Schwefel steht,

Y für Sauerstoff oder Schwefel steht,

$R^1$ für Wasserstoff oder für geradkettiges oder verzweigtes Alkyl mit 1 bis 4 Kohlenstoffatomen steht,

$R^2$ für geradkettiges oder verzweigtes Alkyl mit 1 bis 4 Kohlenstoffatomen steht,

$R^3$ für geradkettiges oder verzweigtes Alkyl mit 1 bis 4 Kohlenstoffatomen oder für geradkettiges oder veriweigtes Alkenyl mit 2 bis 5 Kohlenstoffatomen steht,

$R^4$ für gegebenenfalls ein- bis dreifach, gleich oder verschieden substituiertes Cyclohexyl steht, wobei als Substituenten infrage kommen: Methyl, Ethyl oder Isopropyl oder

für jeweils gegebenenfalls ein- bis dreifach, gleich oder verschieden substituiertes Phenyl oder Naphthyl steht, wobei als Substituenten infrage kommen: Fluor, Chlor, Brom, Cyano,

Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Difluormethyl, Trifluormethoxy, Trifluormethylthio, Methoxycarbonyl, Ethoxycarbonyl, Propoxycarbonyl, Dimethylamino, N-Acetamido, Methoximinomethyl, Methoximinoethyl, Ethoximinomethyl, Ethoximinoethyl, Dioxymethylen, Difluordioxymethylen, Tetrafluordioxyethylen oder gegebenenfalls ein- bis dreifach, gleich oder verschieden durch Fluor, Chlor, Brom, Methyl und/oder Ethyl substituiertes Phenyl,

$R^5$ für geradkettiges oder verzweigtes Alkyl mit 1 bis 4 Kohlenstoffatomen steht oder auch für Wasserstoff steht, für den Fall, daß nicht gleichzeitig A für $-CH_2-CH_2-$ und $R^4$ für unsubstituiertes Phenyl steht und

$R^6$ für Cyano oder für geradkettiges oder verzweigtes Alkyl mit 1 bis 4 Kohlenstoffatomen steht oder auch für Wasserstoff steht, für den Fall, daß A gleichzeitig für einen verzweigten Alkandiylrest der Formel

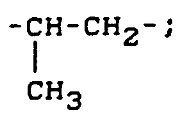 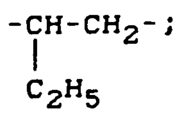 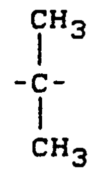 oder 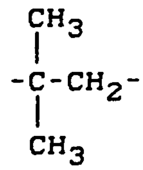

steht,
oder

$R^5$ und $R^6$ gemeinsam für einen zweifach verknüpften Alkandiylrest mit 4 bis 5 Kohlenstoffatomen stehen.

Ganz besonders bevorzugt sind Verbindungen der Formel (I), bei welchen

A für einen Rest der Formel
$-CH_2-CH_2-$; $-CH_2-CH_2-CH_2-$;

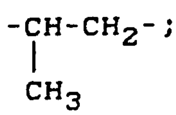 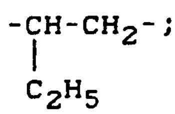 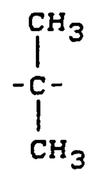

oder 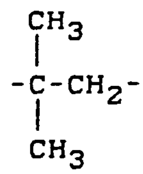

steht,

X für Sauerstoff steht,

Y für Sauerstoff steht,

$R^1$ für Wasserstoff oder für Methyl steht,

$R^2$ für Methyl steht,

$R^3$ für Methyl, Ethyl, n- oder i-Propyl, n-, i-, s- oder t-Butyl oder für Vinyl, Allyl, n- oder i-Butenyl steht,

$R^4$ für gegebenenfalls ein- bis dreifach durch Methyl und/oder Ethyl substituiertes Cyclohexyl steht, oder
für gegebenenfalls ein- bis dreifach, gleich oder verschieden substituiertes Phenyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Difluormethyl, Trifluormethoxy, Trifluormethylthio, Methoxycarbonyl, Ethoxycarbonyl, Propoxycarbonyl, Dimethylamino, N-Acetamido, Dioxymethylen, Difluordioxymethylen, Tetrafluordioxyethylen oder gegebenenfalls ein- bis dreifach, gleich oder verschieden durch Fluor, Chlor, Brom, Methyl und/oder Ethyl substituiertes Phenyl,

$R^5$ für geradkettiges oder verzweigtes Alkyl mit 1 bis 4 Kohlenstoffatomen steht oder auch für Wasserstoff steht, für den Fall, daß nicht gleichzeitig A für $-CH_2-CH_2-$ und $R^4$ für unsubstituiertes Phenyl steht und

$R^6$ für Cyano oder für geradkettiges oder verzweigtes Alkyl mit 1 bis 4 Kohlenstoffatomen steht oder auch für Wasserstoff steht, für den Fall, daß A gleichzeitig für einen verzweigten Alkandiylrest der Formel

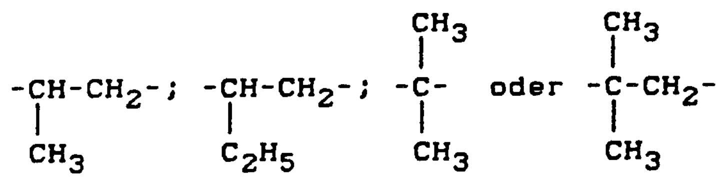

steht,

oder

$R^5$ und $R^6$ gemeinsam für einen zweifach verknüpften Pentandiylrest stehen.

Die oben aufgeführten allgemeinen oder in Vorzugsbereichen aufgeführten Restedefinitionen bzw. Erläuterungen können untereinander, also auch zwischen den jeweiligen Vorzugsbereichen beliebig kombiniert werden.

Verwendet man beispielsweise 1-Chlorcarbonyl-3-dimethylamino-4-methyl-1,2,4-triazolin-5-on und 1-(4-Methylphenyl)-3-aminobutan als Ausgangsstoffe, so läßt sich der Reaktionsablauf des erfindungsgemäßen Verfahrens (a) durch das folgende Formelschema darstellen:

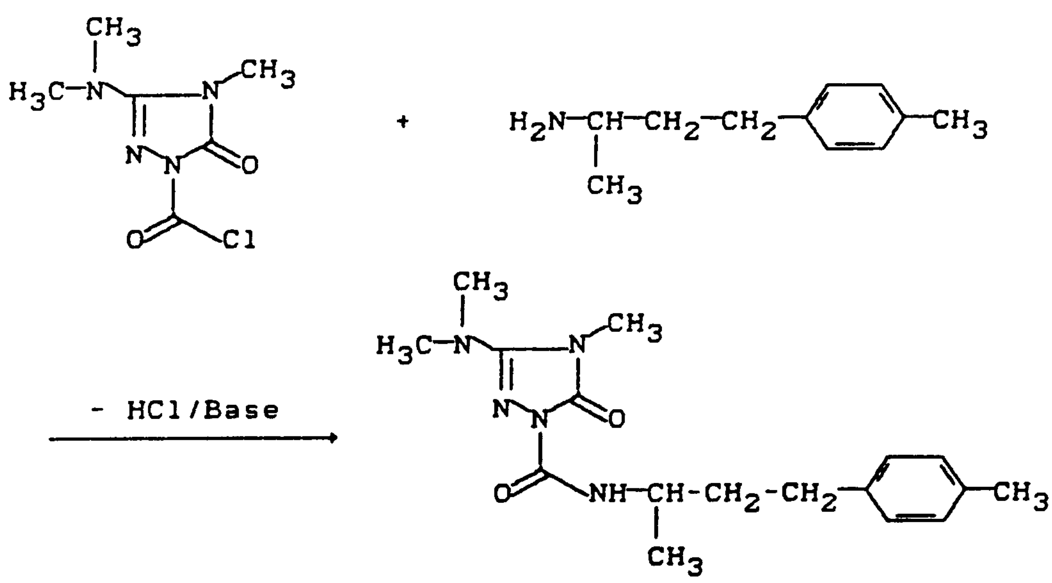


Verwendet man beispielsweise 3-Dimethylamino-4-methyl-1,2,4-triazolin-5-on und 3-Methyl-4-phenyl-2-butylisocyanat als Ausgangsstoffe, so läßt sich der Reaktionsablauf des erfindungsgemäßen Verfahrens (b) durch das folgende Formelschema darstellen:

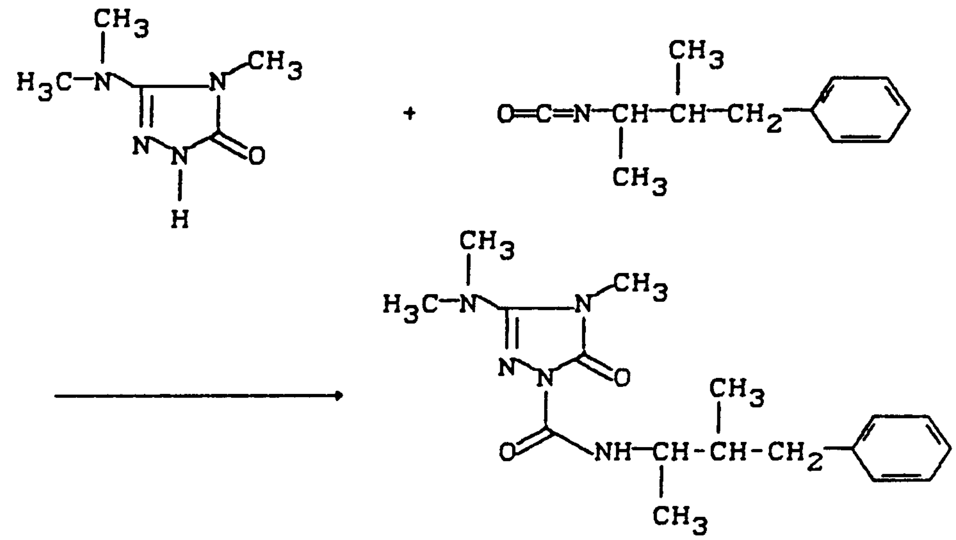

Die zur Durchführung des erfindungsgemäßen Verfahrens (a) als Ausgangsstoffe benötigten 1-Chlor-(thio)-carbonyltriazolinone sind durch die Formel (II) allgemein definiert. In dieser Formel (II) stehen $R^1$, $R^2$, $R^3$, X und Y vorzugsweise für diejenigen Reste, die bereits im Zusammenhang mit der Beschreibung der erfindungsgemäßen Verbindungen der Formel (I) als bevorzugt für diese Substituenten genannt wurden.

Die 1-Chlor-(thio)-carbonyltriazolinone der Formel (II) sind bekannt oder erhältlich in Analogie zu bekannten Verfahren (vergleiche z. B. EP 283 876).

Die zur Durchführung des erfindungsgemäßen Verfahrens (a) weiterhin als Ausgangsstoffe benötigten Amine sind durch die Formel (III) allgemein definiert. In dieser Formel (III) stehen $R^4$, $R^5$, $R^6$ und A vorzugsweise für diejenigen Reste, die bereits im Zusammenhang mit der Beschreibung der erfindungsgemäßen Verbindungen der Formel (I) als bevorzugt für diese Substituenten genannt wurden.

Die Amine der Formel (III) sind bekannt oder erhältlich in Analogie zu bekannten Verfahren (vergleiche z. B. Coll. Czech. Chem. Commun. 35, 2810-2830 [1970]; Synth. Commun. 18, 29-35 [1988]; Angew. Chem. 96, 368-369 [1984]; DE-OS 28 25 961; DE-OS 32 22 152; US 4.906.645; EP 320 898; US 4.695.589; EP 237 305; US 4.374.149; EP 28 105; EP 6 614; Nouv. J. Chim. 1, 243-254 [1977] bzw. CA 87: 151614a).

Die zur Durchführung des erfindungsgemäßen Verfahrens (b) als Ausgangsstoffe benötigten in 1-Stellung unsubstituierten Triazolinone sind durch die Formel (IV) allgemein definiert. In dieser Formel (IV) stehen $R^1$, $R^2$, $R^3$ und X vorzugsweise für diejenigen Reste, die bereits im Zusammenhang mit der Beschreibung der erfindungsgemäßen Verbindungen der Formel (I) als bevorzugt für diese Substituenten genannt wurden.

Die in 1-Stellung unsubstituierten Triazolinone der Formel (IV) sind bekannt oder erhältlich in Analogie zu bekannten Verfahren (vergleiche z. B. EP 283 876).

Die zur Durchführung des erfindungsgemäßen Verfahrens (b) als Ausgangsstoffe benötigten Iso-(thio)-cyanate sind durch die Formel (V) allgemein definiert. In dieser Formel (V) stehen $R^4$, $R^5$, $R^6$, A und Y vorzugsweise für diejenigen Reste, die bereits im Zusammenhang mit der Beschreibung der erfindungsgemäßen Verbindungen der Formel (I) als bevorzugt für diese Substituenten genannt wurden.

Die Iso-(thio)-cyanate der Formel (V) sind teilweise bekannt (vergleiche z. B. Nouv. J. Chim. 1, 243-254 [1977] bzw. CA 87: 151614a; US 4.035.404) oder erhältlich in Analogie zu bekannten Verfahren (vergleiche z. B. Synthesis 1977, 756; Org. Syntheses Coll. Vol. IV, 521 [1963] oder "Organikum" VEB Deutscher Verlag der Wissenschaften Berlin 1981, S. 703), beispielsweise, indem man Amine der Formel (III),

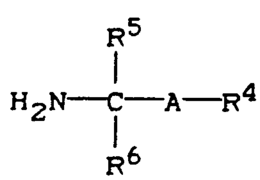

(III)

in welcher

$R^4$, $R^5$, $R^6$ und A die oben angegebene Bedeutung haben,
mit Phosgen oder Thiophosgen gegebenenfalls in Gegenwart eines Verdünnungsmittels wie beispielsweise Toluol oder Chlorbenzol bei Temperaturen zwischen -10°C und +150°C umsetzt.

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester, wie Essigsäuremethylester, Essigsäureethylester oder Basen wie Pyridin.

Das erfindungsgemäße Verfahren (a) kann gegebenenfalls in Gegenwart eines geeigneten Säurebindemittels als Reaktionshilfsmittel durchgeführt werden. Als solche kommen alle üblichen anorganischen oder organischen Basen infrage. Hierzu gehören beispielsweise Erdalkali- oder Alkalimetallhydroxide, wie Natriumhydroxid, Calciumhydroxid, Kaliumhydroxid oder auch Ammoniumhydroxid, Alkalimetallcarbonate, wie Natriumcarbonat, Kaliumcarbonat, Kaliumhydrogencarbonat, Natriumhydrogencarbonat oder Ammoniumcarbonat, Alkali- oder Erdalkalimetallacetate, wie Natriumacetat, Kaliumacetat, Calciumacetat oder Ammoniumacetat, sowie tertiäre Amine, wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Es ist auch möglich, das als Reaktionspartner verwendete Amin der Formel (III) in entsprechendem Überschuß gleichzeitig als Säurebindemittel einzusetzen.

Die Reaktionstemperaturen können bei der Durchführung des erfindungsgemäßen Verfahrens (a) in einem größeren Bereich variiert werden. Im allgemeinen arbeitet man bei Temperaturen zwischen 0°C und +150°C, vorzugsweise bei Temperaturen zwischen +10°C und +80°C.

Zur Durchführung des erfindungsgemäßen Verfahrens (a) setzt man pro Mol an 1-Chlor-(thio)-carbonyltriazolinon der Formel (II) im allgemeinen 1,0 bis 5,0 Mol, vorzugsweise 1,0 bis 2,5 Mol an Amin der Formel (III) und gegebenenfalls 1,0 bis 5,0 Mol, vorzugsweise 1,0 bis 2,5 Mol an als Reaktionshilfsmittel verwendeter Base ein.

Die Reaktionsdurchführung, Aufarbeitung und Isolierung der Reaktionsprodukte erfolgt nach bekannten Verfahren (vergleiche hierzu beispielsweise EP 283 876 oder die Herstellungsbeispiele).

Als Verdünnungsmittel zur Durchführung des erfindungsgemäßen Verfahrens (b) kommen ebenfalls inerte organische Lösungsmittel infrage. Vorzugsweise verwendet man die bei Verfahren (a) genannten Verdünnungsmittel.

Das erfindungsgemäße Verfahren (b) wird vorzugsweise in Gegenwart eines geeigneten basischen Reaktionshilfsmittels durchgeführt. Als solche kommen alle üblichen anorganischen oder organischen Basen infrage. Vorzugsweise verwendet man tertiäre Amine, wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU). Der Zusatz solcher basischer Reaktionshilfsmittel ist jedoch nicht zwingend erforderlich.

Die Reaktionstemperaturen können bei der Durchführung des erfindungsgemäßen Verfahrens (b) in einem größeren Bereich variiert werden. Im allgemeinen arbeitet man bei Temperaturen zwischen 0°C und +150°C, vorzugsweise bei Temperaturen zwischen +40°C und +120°C.

Das erfindungsgemäße Verfahren (b) wird üblicherweise unter Normaldruck durchgeführt. Es ist jedoch auch möglich, insbesondere bei gasförmigen Ausgangsverbindungen, unter erhöhtem Druck zu arbeiten.

Zur Durchführung des erfindungsgemäßen Verfahrens (b) setzt man pro Mol an in 1-Stellung unsubstituiertem Triazolinon der Formel (IV) im allgemeinen 1,0 bis 5,0 Mol, vorzugsweise 1,0 bis 2,5 Mol an Iso-(thio)-cyanat der Formel (V) und gegebenenfalls 1,0 bis 5,0 Mol, vorzugsweise 1,0 bis 2,5 Mol an als Reaktionshilfsmittel verwendeter Base ein.

Die Reaktionsdurchführung, Aufarbeitung und Isolierung der Reaktionsprodukte erfolgt nach bekannten Verfahren (vergleiche hierzu beispielsweise EP 283 876 oder die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga,

Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung von mono- und dikotylen Unkräutern insbesondere in monokotylen Kulturen wie beispielsweise Weizen einsetzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid; als feste Trägerstoffe kommen infrage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische, pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage. Auch Mischungen mit 2,4-Dichlorphenoxyessigsäure (2,4-D); 4-(2,4-Dichlorphenoxy)-buttersäure (2,4-DB); 2,4-Dichlorphenoxypropionsäure (2,4-DP); (2-Methyl-4-chlorphenoxy)-essigsäure (MCPA); 4-(4-Chlor-2-methyl)-phenoxybuttersäure (MCPB); [(4-Amino-3,5-dichlor-6-fluor-2-pyridinyl)-oxy]-essigsäure bzw. deren 1-Methylheptylester (FLUROXYPYR); 2-[4-(2,4-Dichlorphenoxy)-phenoxy]-propionsäure, deren Methyl- oder deren Ethylester (DICLOFOP); 2-{4-[(6-Chlor-2-benzoxazolyl)-oxy]-phenoxy}-propansäure, deren Methyl- oder deren Ethylester (FENOXAPROP); N-(1-Ethylpropyl)-3,4-dimethyl-2,6-dinitroanilin (PENDIMETHALIN); Methyl-5-(2,4-dichlorphenoxy)-2-nitrobenzoat (BIFENOX); N,N-Dimethyl-N'-(3-chlor-4-methylphenyl)-harnstoff (CHLORTOLURON); N,N-Dimethyl-N'-(4-isopropylphenyl)-harnstoff (ISOPROTURON); Methyl-2-[4,5-dihydro-4-methyl-4-(1-methylethyl)-5-oxo-1H-imidazol-2-yl]-4(5)-methylbenzoat (IMAZAMETHABENZ); 3,5-Dibrom-4-hydroxy-benzonitril (BROMOXYNIL); 3,5-Diiod-4-hydroxybenzonitril (IOXYNIL); N-Methyl-2-(1,3-benzthiazol-2-yloxy)-acetanilid (MEFENACET); 2-Chlor-N-{[(4-methoxy-6-methyl-1,3,5-triazin-2-yl)-amino]-carbonyl}-benzolsulfonamid (CHLORSULFURON); 2-{[[((4-Methoxy-6-methyl-1,3,5-triazin-2-yl)-amino)-carbonyl]-amino]-sulfonyl}-benzoesäure oder deren Methylester (METSULFURON); 3-[[[[(4-Methoxy-6-methyl-1,3,5-triazin-2-yl)-amino]-carbonyl]-amino]-sulfonyl]-thiophen-2-carbonsäure-methylester (THIAMETURON); N,N-Diisopropyl-S-(2,3,3-trichlorallyl)-thiolcarbamat (TRIALLATE); 2-Chlor-4-ethylamino-6-isopropylamino-1,3,5-triazin (ATRAZIN); 2-Chlor-4-ethylamino-6-(3-cyanapropylamino)-1,3,5-triazin (CYANAZIN); 4-Ethylamino-2-t-butylamino-6-methylthio-s-triazin (TERBUTRYNE); 4-Amino-6-t-butyl-3-ethylthio-1,2,4-triazin-5(4H)-on (ETHIOZIN); 3-Isopropyl-2,1,3-benzothiadiazin-4-on-2,2-dioxid (BENTAZON); 0-(6-Chlor-3-phenyl-pyridazin-4-yl)-S-octylthiocarbonat (PYRIDATE) sind gegebenenfalls von Vorteil. Einige Mischungen zeigen überraschenderweise auch synergistische Wirkungen.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele:

Beispiel 1:

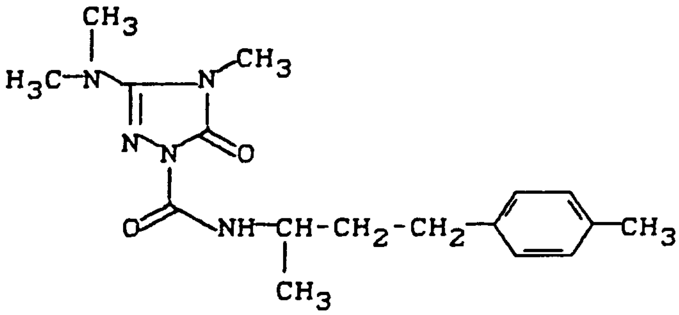

(Verfahren a)

Zu 5,1 g (0,025 Mol) 1-Chlorcarbonyl-3-dimethylamino-4-methyl-1,2,4-triazolin-5-on in 100 ml Acetonitril tropft man unter Rühren bei Raumtemperatur eine Lösung von 4,08 g (0,025 Mol) 4-(4-Methylphenyl)-2-butylamin (vergleiche z. B. DE-OS 32 22 152) und 2,53 g (0,025 Mol) Triethylamin in 50 ml Acetonitril. Nach beendeter Zugabe rührt man 4 Stunden bei Raumtemperatur, filtriert dann ausgefallenes Triethylamin-Hydrochlorid ab, engt im Vakuum ein, nimmt den öligen Rückstand in 150 ml Dichlormethan auf, wäscht dreimal mit jeweils 50 ml Wasser, trocknet über Natriumsulfat und entfernt das Lösungsmittel im Vakuum. Der Rückstand wird über Kieselgel chromatographiert (Laufmittel: Cyclohexan/Essigester 1:1).

Man erhält 3,5 g (42 % der Theorie) an 1-[4-(4-Methylphenyl)-but-2-ylaminocarbonyl]-3-dimethylamino-4-methyl-1,2,4-triazolin-5-on als Öl.

$^1$H-NMR ($CDCl_3$/Tetramethylsilan):

$\delta$ = 1,23-1,25; 1,75-1,85; 2,3; 2,6-2,7; 2,85; 3,25; 4,0-4,15; 7,05; 7,85-7,9 ppm.

Beispiel 2:

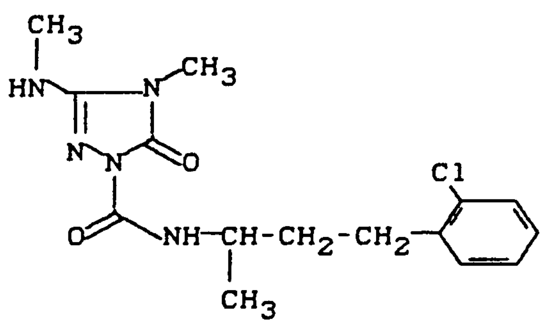

(Verfahren a)

Zu 4,76 g (0,025 Mol) 1-Chlorcarbonyl-3-methylamino-4-methyl-1,2,4-triazolin-5-on in 100 ml Acetonitril tropft man unter Rühren bei Raumtemperatur eine Lösung von 4,58 g (0,025 Mol) 4-(2-Chlorphenyl)-2-butylamin (Herstellung vergleiche z. B. EP 6 614) und 2,53 g (0,025 Mol) Triethylamin in 50 ml Acetonitril. Nach beendeter Zugabe rührt man 4 Stunden bei Raumtemperatur, filtriert dann ausgefallenes Triethylamin-Hydrochlorid ab und engt im Vakuum ein. Der feste Rückstand wird mit Wasser verrührt und aus Cyclohexan/Essigester (1:1) umkristallisiert.

Man erhält 5,8 g (69 % der Theorie) an 1-[4-(2-Chlorphenyl)-but-2-ylaminocarbonyl]-3-methylamino-4-methyl-1,2,4-triazolin-5-on vom Schmelzpunkt 133°C bis 135°C.

Beispiel 3:

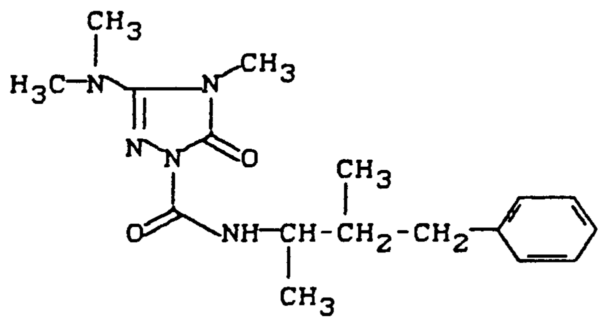

(Verfahren b)

Zu 3,55 g (0,025 Mol) 3-Dimethylamino-4-methyl-1,2,4-triazolin-5-on in 80 ml Acetonitril gibt man bei Raumtemperatur 4,9 g (0,026 Mol) 4-Phenyl-3-methyl-but-2-ylisocyanat (Herstellung vergleiche z. B. Nouv. J. Chim. 1, 243-254 [1977] bzw. CA 87:151614a sowie DE-OS 32 22 152) und 3 Tropfen 1,8-Diazabicyclo-[5.4.0]-undec-7-en (DBU), rührt 6 Stunden bei Raumtemperatur, engt im Vakuum ein, und chromatographiert den Rückstand über Kieselgel (Laufmittel: Cyclohexan/Essigester 1:1).

Man erhält 5,4 g (65 % der Theorie) an 1-(4-Phenyl-3-methyl-but-2-ylaminocarbonyl)-3-dimethylamino-4-methyl-1,2,4-triazolin-5-on als Öl.

$^1$H-NMR ($CDCl_3$/Tetramethylsilan):

$\delta$ = 0,85-0,95; 1,23-1,25; 1,9-2,0; 2,3-2,4; 2,85; 3,25; 4,1-4,2; 7,15-7,25; 8,0-8,1 ppm.

Beispiel 4:

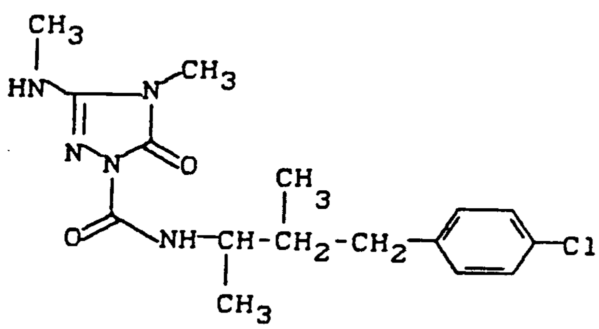

(Verfahren b)

Zu 2,56 g (0,02 Mol) 3-Methylamino-4-methyl-1,2,4-triazolin-5-on in 80 ml Acetonitril gibt man bei Raumtemperatur 4,5 g (0,02 Mol) 4-(4-Chlorphenyl)-3-methylbut-2-ylisocyanat (Herstellung vergleiche z. B. Nouv. J. Chim. 1, 243-254 [1977] bzw. CA 87:151614a sowie DE-OS 32 22 152) und 3 Tropfen 1,8-Diazabicyclo-[5.4.0]-undec-7-en (DBU), rührt 2 Stunden bei Rückflußtemperatur, engt im Vakuum ein und chramatographiert den Rückstand über Kieselgel (Laufmittel: Cyclohexan/Ethanol 1:1).

Man erhält 3,4 g (48 % der Theorie) an 1-[4-(4-Chlorphenyl)-3-methyl-but-2-ylaminocarbonyl]-3-methylamino-4-methyl-1,2,4-triazolin-5-on vom Schmelzpunkt 222 bis 224°C.

In entsprechender Weise und gemäß den allgemeinen Angaben zur Herstellung erhält man die in der nachfolgenden Tabelle*)aufgeführten substituierten Triazolinone der allgemeinen Formel (I):

*) Die $^1$H-NMR-Spektren wurden in Deuterochloraform ($CDCl_3$) mit Tetramethylsilan (TMS) als innerem Standard aufgenommen. Angegeben ist die chemische Verschiebung als $\delta$-Wert in ppm.

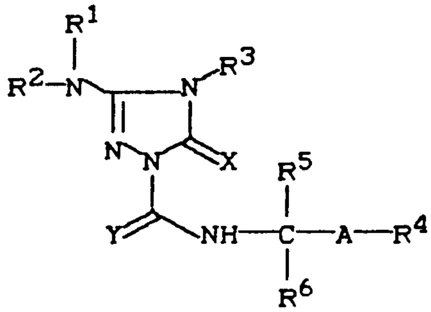

(I)

$R^4$ =

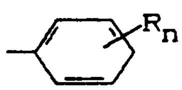

| Bsp.Nr. | $-N(R^1R^2)$ | $R^3$ | $R_n$ | $R^5$ | $R^6$ | A | X | Y | $^1H$-NMR*) oder Fp[°C] |
|---|---|---|---|---|---|---|---|---|---|
| 5 | $-N(CH_3)_2$ | $CH_3$ | $4-t-C_4H_9$ | H | H | $-CH-CH_2-$<br>$\vert$<br>$CH_3$ | O | O | 0,9-0,95; 1,3; 2,85; 3,25 |
| 6 | $-N(CH_3)_2$ | $CH_3$ | H | $CH_3$ | $CH_3$ | $-CH_2-CH_2-$ | O | O | 1,45; 2,0-2,1; 2,6-2,7; 2,85; 3,25; 7,1-7,3 |
| 7 | $-N(CH_3)_2$ | $CH_3$ | 4-Cl | H | $CH_3$ | $-CH-CH_2-$<br>$\vert$<br>$CH_3$ | O | O | 1,2-1,25; 3,25; 7,1-7,25 |
| 8 | $-N(CH_3)_2$ | $CH_3$ | 4-Cl | H | H | $-CH-CH_2-$<br>$\vert$<br>$CH_3$ | O | O | 0,9-0,95; 2,85; 7,1-7,25 |
| 9 | $-N(CH_3)_2$ | $CH_3$ | 4-Cl | H | $CH_3$ | $-CH-CH_2-$<br>$\vert$<br>$C_2H_5$ | O | O | 2,85; 3,25; 4,05-4,15; 7,1-7,25 |
| 10 | $-N(CH_3)_2$ | $CH_3$ | 4-F | H | $CH_3$ | $-CH-CH_2-$<br>$\vert$<br>$CH_3$ | O | O | 1,2-1,25; 2,85; 6,9-7,15 |
| 11 | $-N(CH_3)_2$ | $CH_3$ | 2,4-di-Cl | $CH_3$ | $CH_3$ | $-CH_2-CH_2-$ | O | O | 1,45; 2,0-2,1; 2,7-2,8; 2,85 |
| 12 | $-N(CH_3)_2$ | $i-C_3H_7$ | 4-Cl | H | $CH_3$ | $-CH-CH_2-$<br>$\vert$<br>$CH_3$ | O | O | 1,5-1,55; 2,85; 4,1-4,2; 7,1-7,25; 8,05-8,1 |
| 13 | $-N(CH_3)_2$ | $CH_3$ | H | $CH_3$ | $C_2H_5$ | $-CH_2-CH_2-$ | O | O | 0,9-0,95; 2,6-2,65; 2,85; 7,9 |

| Bsp.Nr. | $-N(R^1R^2)$ | $R^3$ | $R_n$ | $R^5$ | $R^6$ | A | X | Y | $^1H$-NMR*) oder Fp[°C] |
|---|---|---|---|---|---|---|---|---|---|
| 14 | $-N(CH_3)_2$ | $CH_3$ | H | $C_2H_5$ | $C_2H_5$ | $-CH_2-CH_2-$ | O | O | Fp.: 94-95° C |
| 15 | $-N(CH_3)_2$ | $CH_3$ | 4-F | $CH_3$ | $CH_3$ | $-CH_2-CH_2-$ | O | O | Fp.: 95-96° C |
| 16 | $-N(CH_3)_2$ | $CH_3$ | $4-CH_3$ | $CH_3$ | $CH_3$ | $-CH_2-CH_2-$ | O | O | Fp.: 98° C |
| 17 | $-N(CH_3)_2$ | $CH_3$ | 3-Cl | $CH_3$ | $CH_3$ | $-CH_2-CH_2-$ | O | O | 1,45; 2,05-2,1; 2,85; 3,25; 4,0-4,1 |
| 18 | $-N(CH_3)_2$ | $CH_3$ | 4-Cl | $CH_3$ | $CH_3$ | $-CH_2-CH_2-$ | O | O | Fp.: 81° C |
| 19 | $-N(CH_3)_2$ | $CH_3$ | 4-Cl | H | $CH_3$ | $-CH_2-CH_2-$ | O | O | 1,25; 2,6-2,7; 2,85; 3,25; 4,0-4,1 |
| 20 | $-N(CH_3)_2$ | $CH_3$ | $3,4-di-CH_3$ | H | $CH_3$ | $-CH_2-CH_2-$ | O | O | 1,25; 2,85; 3,25; 4,0-4,1; 7,0-7,1; 7,3-7,35 |
| 21 | $-N(CH_3)_2$ | $CH_3$ | 2-Cl | H | $CH_3$ | $-CH_2-CH_2-$ | O | O | 1,25-1,3; 1,3-1,4; 2,85; 3,25 |
| 22 | $-N(CH_3)_2$ | $CH_3$ | $4-CH_3$ | H | $CH_3$ | $-C(CH_3)_2-CH_2-$ | O | O | 2,3; 2,85; 3,25; 7,0-7,1 |

| Bsp.Nr. | $-N(R^1R^2)$ | $R^3$ | $R_n$ | $R^5$ | $R^6$ | A | X | Y | $^1H$-NMR*)<br>oder Fp[°C] |
|---|---|---|---|---|---|---|---|---|---|
| 23 | $-N(CH_3)_2$ | $CH_3$ | $4-OCH_3$ | H | $CH_3$ | $-CH_2-CH_2-$ | O | O | 1,25; 1,75-1,85;<br>2,85; 3,75 |
| 24 | $-N(CH_3)_2$ | $CH_3$ | $4-t-C_4H_9$ | H | $CH_3$ | $-CH_2-CH_2-$ | O | O | 1,3; 2,6-2,7;<br>2,85; 4,0-4,1 |
| 25 | $-N(CH_3)_2$ | $CH_3$ | 2-Cl | H | $CH_3$ | $-CH-CH_2-$<br>\|<br>$CH_3$ | O | O | 1,2-1,25; 2,85;<br>3,25; 7,0-7,25 |
| 26 | $-N(CH_3)_2$ | $CH_3$ | 4-F | H | $CH_3$ | $-CH_2-CH_2-$ | O | O | 1,25; 1,8-1,9;<br>2,6-2,7; 3,25 |
| 27 | $-N(CH_3)_2$ | $CH_3$ | $3-CH_3$ | H | $CH_3$ | $-CH-CH_2-$<br>\|<br>$CH_3$ | O | O | 2,3; 2,85; 3,25;<br>8,0-8,1 |
| 28 | $-N(CH_3)_2$ | $CH_3$ | $2-CH_3$ | H | $CH_3$ | $-CH-CH_2-$<br>\|<br>$CH_3$ | O | O | 2,85; 3,25;<br>7,05-7,15;<br>8,0-8,1 |
| 29 | $-N(CH_3)_2$ | $CH_3$ | $3-CF_3$ | H | $CH_3$ | $-CH_2-CH_2$ | O | O | 1,4; 1,85-1,9;<br>2,85; 4,05-4,15 |
| 30 | $-N(CH_3)_2$ | $CH_3$ | $3-OCH_3$ | H | $CH_3$ | $-CH-CH_2-$<br>\|<br>$CH_3$ | O | O | 2,85; 3,25; 3,8;<br>4,1-4,15;<br>8,0-8,05 |
| 31 | $-N(CH_3)_2$ | $CH_3$ | $3-OC_2H_5$ | H | $CH_3$ | $-CH-CH_2-$<br>\|<br>$CH_3$ | O | O | 2,85; 3,25;<br>3,95-4,05;<br>8,0-8,1 |

| Bsp.Nr. | $-N(R^1R^2)$ | $R^3$ | $R_n$ | $R^5$ | $R^6$ | A | X | Y | $^1H$-NMR*) oder Fp[°C] |
|---|---|---|---|---|---|---|---|---|---|
| 32 | $-N(CH_3)_2$ | $CH_3$ | $3-O-n-C_3H_7$ | H | $CH_3$ | $-CH(CH_3)-CH_2-$ | O | O | 2,85; 3,25; 3,85-3,95; 8,0-8,1 |
| 33 | $-N(CH_3)_2$ | $CH_3$ | $2-NH-CO-CH_3$ | H | $CH_3$ | $-CH_2-CH_2-$ | O | O | 2,15; 2,85; 4,0-4,1; 7,55 |
| 34 | $-N(CH_3)_2$ | $CH_3$ | 3,4-di-$CH_3$ | H | $CH_3$ | $-CH(CH_3)-CH_2-$ | O | O | 2,2; 2,85; 6,9-7,05; 8,0-8,05 |
| 35 | $-N(CH_3)_2$ | $CH_3$ | $3-CF_3$ | H | $CH_3$ | $-CH(CH_3)-CH_2-$ | O | O | 1,25; 2,85; 3,3; 7,4-7,45 |
| 36 | $-N(CH_3)_2$ | $CH_3$ | $2-OC_2H_5$ | H | $CH_3$ | $-CH(CH_3)-CH_2-$ | O | O | 1,25-1,3; 2,85; 4,0-4,05 |
| 37 | $-N(CH_3)_2$ | $CH_3$ | $4-N(CH_3)_2$ | $CH_3$ | $CH_3$ | $-CH_2-CH_2-$ | O | O | 1,45; 2,0-2,1; 2,85; 2,9; 3,25 |
| 38 | $-NH-CH_3$ | $CH_3$ | 4-Cl | H | $CH_3$ | $-CH_2-CH_2-$ | O | O | Fp.: 120-121° C |
| 39 | $-NH-CH_3$ | $CH_3$ | 3-Cl | H | $CH_3$ | $-CH_2-CH_2-$ | O | O | Fp.: 114-116° C |
| 40 | $-NH-CH_3$ | $CH_3$ | $3-OCH_3$ | H | $CH_3$ | $-CH(CH_3)-CH_2-$ | O | O | Fp.: 124-126° C |

| Bsp.Nr. | $-N(R^1R^2)$ | $R^3$ | $R_n$ | $R^5$ | $R^6$ | A | X | Y | $^1H$-NMR*) oder Fp[°C] |
|---|---|---|---|---|---|---|---|---|---|
| 41 | $-NH-CH_3$ | $CH_3$ | $4-CH_3$ | H | $CH_3$ | $-CH_2-CH_2-$ | O | O | Fp.: 134-136° C |
| 42 | $-NH-CH_3$ | $CH_3$ | $3-CF_3$ | H | $CH_3$ | $-CH_2-CH_2-$ | O | O | Fp.: 128-130° C |
| 43 | $-NH-CH_3$ | $CH_3$ | $4-t-C_4H_9$ | H | $CH_3$ | $-CH_2-CH_2-$ | O | O | Fp.: 136-138° C |
| 44 | $-NH-CH_3$ | $CH_3$ | 3,4-di-Cl | H | $CH_3$ | $-CH_2-CH_2-$ | O | O | Fp.: 95-97° C |
| 45 | $-NH-CH_3$ | $CH_3$ | $4-OCH_3$ | H | $CH_3$ | $-CH_2-CH_2-$ | O | O | Fp.: 112-114° C |
| 46 | $-NH-CH_3$ | $CH_3$ | $3,4-di-CH_3$ | H | $CH_3$ | $-\underset{CH_3}{CH}-CH_2-$ | O | O | Fp.: 143-144° C |
| 47 | $-NH-CH_3$ | $CH_3$ | $2-OC_2H_5$ | H | $CH_3$ | $-\underset{CH_3}{CH}-CH_2-$ | O | O | Fp.: 119-121° C |
| 48 | $-NH-CH_3$ | $CH_3$ | $3-OC_2H_5$ | H | $CH_3$ | $-\underset{CH_3}{CH}-CH_2-$ | O | O | Fp.: 128-130° C |
| 49 | $-NH-CH_3$ | $CH_3$ | $3-CF_3$ | H | $CH_3$ | $-\underset{CH_3}{CH}-CH_2-$ | O | O | Fp.: 134-135° C |
| 50 | $-\underset{CH_3}{N}-iC_3H_7$ | $CH_3$ | 4-Cl | H | $CH_3$ | $-CH_2-CH_2$ | O | O | |

| Bsp.Nr. | $-N(R^1R^2)$ | $R^3$ | $R_n$ | $R^5$ | $R^6$ | A | X | Y | $^1H$-NMR*) oder Fp[°C] |
|---|---|---|---|---|---|---|---|---|---|
| 51 | $-N(CH_3)_2$ | $CH_3$ | $4-OC_2H_5$ | H | $CH_3$ | $-CH_2-CH_2-$ | O | O | 1,35-1,40; 2,6-2,7; 2,85; 3,95-4,05 |
| 52 | $-N(CH_3)_2$ | $CH_3$ | 2-Cl | H | H | $-CH-CH_2-$<br>$CH_3$ | O | O | 0,95-0,97; 2,85; 4,13-4,17 |
| 53 | $-N(CH_3)_2$ | $CH_3$ | $3,4-O-CH_2-O-$ | H | $CH_3$ | $-CH_2-CH_2-$ | O | O | 1,75-1,85; 2,55-2,65; 2,85; 5,9; 6,6-6,7 |
| 54 | $-N(CH_3)_2$ | $CH_3$ | 2,3-CH=CH-CH=CH- | H | $CH_3$ | $-CH_2-CH_2-$ | O | O | 1,92-2,05; 2,85; 4,15-4,25; 7,35-7,50 |
| 55 | $-NH-CH_3$ | $CH_3$ | $4-OC_2H_5$ | H | $CH_3$ | $-CH_2-CH_2-$ | O | O | Fp.: 132-133°C |
| 56 | $-N-CH_3$<br>$C_2H_5$ | $CH_3$ | 4-Cl | H | $CH_3$ | $-CH_2-CH_2-$ | O | O | 1,15-1,22; 1,25-1,27; 1,75-1,85; 2,85; 7,8-7,9 |
| 57 | $-N-CH_3$<br>$C_2H_5$ | $CH_3$ | 4-Cl | H | $CH_3$ | $-CH-CH_2-$<br>$CH_3$ | O | O | 0,9-0,95; 2,85; 7,95-8,05 |
| 58 | $-N-CH_3$<br>$C_2H_5$ | $CH_3$ | H | H | $CH_3$ | $-CH-CH_2-$<br>$CH_3$ | O | O | 2,85; 3,10-3,20; 4,0-4,15; 7,15-7,30 8,05-8,10 |

| Bsp.-Nr. | $-N(R^1R^2)$ | $R^3$ | $R^4$ | $R^5$ | $R^6$ | A | X | Y | $^1$H-NMR*) oder Fp/°C |
|---|---|---|---|---|---|---|---|---|---|
| 59 | $-N(CH_3)_2$ | $CH_3$ | 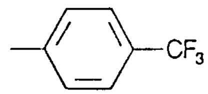 | H | $CH_3$ | $-CH_2-CH_2-$ | O | O | 1,25-1,27; 1,75-1,8; 2,85; 4,05-4,15; 7,3-7,35; 7,5-7,55 |
| 60 | $-N(CH_3)_2$ | $CH_3$ | 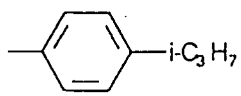 | H | H | 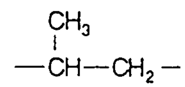 | O | O | 0,92-0,95; 1,2-1,25; 2,0-2,1; 2,85; 3,25; 7,05-7,15 |
| 61 | $-NH-CH_3$ | $CH_3$ | 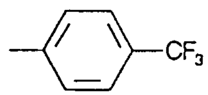 | H | $CH_3$ | $-CH_2-CH_2-$ | O | O | 1,25-1,27; 1,8-1,9; 2,7-2,8; 2,95-3,0; 3,15; 4,0-4,1 |
| 62 | $-NH-CH_3$ | $CH_3$ | 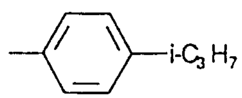 | H | H | 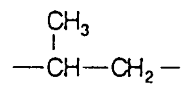 | O | O | 0,9-0,95; 1,2 - 1,25; 2,9-2,95; 3,15; 4,7-4,75 |
| 63 | $-N(CH_3)_2$ | $CH_3$ | 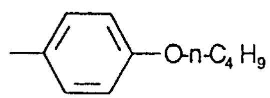 | H | $CH_3$ | 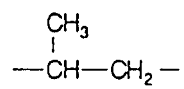 | O | O | 0,95-1,0; 1,2-1,25; 1,45-1,55; 2,85; 3,9-3,95 |
| 64 | $-N(CH_3)_2$ | $CH_3$ | 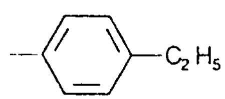 | H | $CH_3$ | $-CH_2-CH_2-$ | O | O | 1,18-1,23; 1,25-1,27; 1,8-1,9; 2,85; 7,1 |

| Bsp.-Nr. | $-N(R^1R^2)$ | $R^3$ | $R^4$ | $R^5$ | $R^6$ | A | X | Y | $^1$H-NMR*) oder Fp/°C |
|---|---|---|---|---|---|---|---|---|---|
| 65 | $-N(CH_3)_2$ | $CH_3$ | 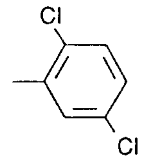 | $CH_3$ | $CH_3$ | $-CH_2-CH_2-$ | O | O | 1,5; 2,0-2,1; 2,7-2,8; 2,85; 3,25; 8,0 |
| 66 | $-NH-CH_3$ | $CH_3$ | 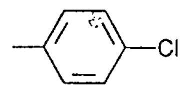 | H | H | 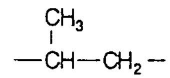 | O | O | Fp 115-116°C |
| 67 | $-N(CH_3)_2$ | $CH_3$ | 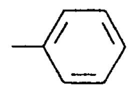 | $CH_3$ | CN | $-CH_2-CH_2-$ | O | O | Fp 172-174°C |
| 68 | $-N(CH_3)_2$ | $C_2H_5$ | 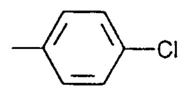 | H | $CH_3$ | $-CH_2-CH_2-$ | O | O | 1,35-1,4; 1,8-1,9; 2,88; 3,68-3,75; 7,1-7,25 |
| 69 | $-N(CH_3)_2$ | $C_2H_5$ | 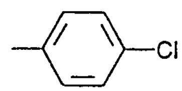 | H | $CH_3$ | 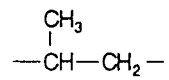 | O | O | 1,3-1,33; 2,85; 3,7-3,8; 8,0-8,05 |
| 70 | $-N(CH_3)_2$ | $C_2H_5$ | 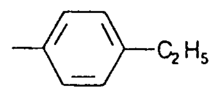 | H | $CH_3$ | $-CH_2-CH_2-$ | O | O | 1,2-1,38; 1,8-1,9; 2,88; 3,7-3,78; 7,1; 7,87-8,93 |
| 71 | $-N(CH_3)_2$ | $C_2H_5$ | 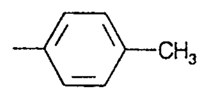 | H | $CH_3$ | $-CH_2-CH_2-$ | O | O | 1,24-1,27; 2,3; 2,85; 3,7-3,78; 7,1; 7,9-7,95 |

| Bsp.-Nr. | $-N(R^1R^2)$ | $R^3$ | $R^4$ | $R^5$ | $R^6$ | A | X | Y | $^1H$-NMR*) oder Fp/°C |
|---|---|---|---|---|---|---|---|---|---|
| 72 | $-N(CH_3)_2$ | $C_2H_5$ | 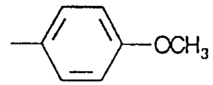 | H | $CH_3$ | $-CH_2-CH_2-$ | O | O | 1,25-1,28; 2,6-2,7; 2,85; 3,75; 7,85-7,93 |
| 73 | $-N(CH_3)_2$ | $C_2H_5$ | 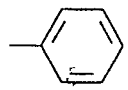 | $CH_3$ | CN | $-CH_2-CH_2-$ | O | O | Fp 166-168°C |
| 74 | $-N(CH_3)_2$ | $CH_3$ | 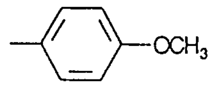 | $CH_3$ | CN | $-CH_2-CH_2-$ | O | O | Fp 110-111°C |
| 75 | $-N(CH_3)_2$ | $CH_3$ | 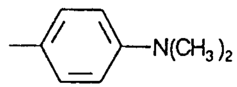 | H | $CH_3$ | $-CH_2-CH_2-$ | O | O | 1,2-1,45; 2,55-2,7; 2,85; 2,9; 7,85-7,9 |
| 76 | $-N(CH_3)_2$ | $CH_3$ | 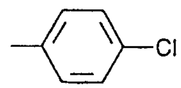 | H | H | 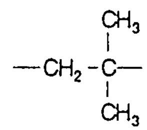 | O | O | Fp 86-88°C |
| 77 | $-N(CH_3)_2$ | $CH_3$ | 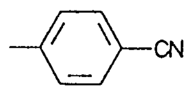 | $CH_3$ | $CH_3$ | $-CH_2-CH_2-$ | O | O | Fp 112-113°C |
| 78 | $-N(CH_3)_2$ | $CH_3$ | 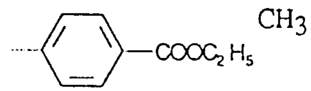 | $CH_3$ | $CH_3$ | $-CH_2-CH_2-$ | O | O | Fp 72-74°C |

| Bsp.-Nr. | $-N(R^1R^2)$ | $R^3$ | $R^4$ | $R^5$ | $R^6$ | A | X | Y | $^1H$-NMR*) oder Fp/°C |
|---|---|---|---|---|---|---|---|---|---|
| 79 | $-N(CH_3)_2$ | $CH_3$ | 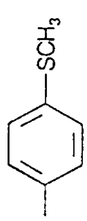 | H | $CH_3$ | $-CH_2-CH_2-$ | O | O | 1,23-1,26; 2,45; 2,88; 3,27 |
| 80 | $-N(CH_3)_2$ | $CH_3$ | 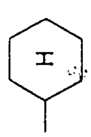 | H | $i-C_4H_9$ | $-CH_2-CH_2-$ | O | O | 0,9-0,95; 2,85; 3,25; 3,93-4,03 |
| 81 | $-N(CH_3)_2$ | $CH_3$ | 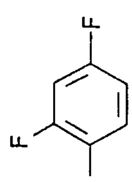 | H | $CH_3$ | $-CH_2-CH_2-$ | O | O | 1,25-1,28; 1,78-1,88; 2,88; 3,28; 7,85-7,92 |
| 82 | $-N(CH_3)_2$ | $CH_3$ | 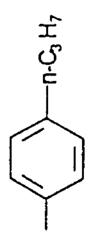 | H | $CH_3$ | $-CH_2-CH_2-$ | O | O | 0,9-0,95; 1,25-1,28; 1,55-1,65; 2,5-2,55; 2,38; 3,27; 4,0-4,1 |
| 83 | $-N(CH_3)_2$ | $CH_3$ | 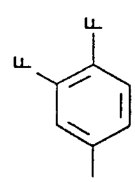 | H | $CH_3$ | $-CH_2-CH_2-$ | O | O | 1,25-1,27; 1,8-1,9; 2,6-2,7; 2,88; 3,28 |
| 84 | $-N(CH_3)_2$ | $CH_3$ | 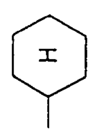 | $CH_3$ | $CH_3$ | $-CH_2-CH_2-$ | O | O | 1,38; 2,85; 3,25 |

Anwendungsbeispiele:

In den folgenden Anwendungsbeispielen wurde die nachstehend aufgeführte Verbindung als Vergleichsubstanz eingesetzt:

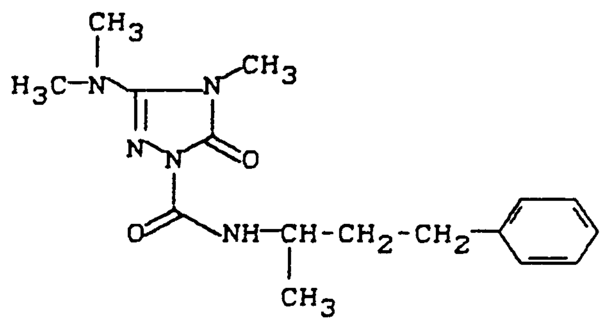

(A)

1-(4-Phenyl-2-butylaminocarbonyl)-3-dimethylamino-4-methyl-1,2,4-triazolin-5-on (bekannt aus DE-OS 37 09 574).

Beispiel A:

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit bei vergleichbarer Nutzpflanzenselektivität gegenüber dem Stand der Technik zeigen in diesem Test z.B. die Verbindungen gemäß den Herstellungsbeispielen 3 und 6.

Beispiel B:

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

Eine deutliche Überlegenheit in der Wirksamkeit bei vergleichbarer Nutzpflanzenselektivität gegenüber dem Stand der Technik zeigen in diesem Test z.B. die Verbindungen gemäß den Herstellungsbeispielen 3, 5 und 6.

**Patentansprüche**

**1.** Substituierte Triazolinone der allgemeinen Formel (I),

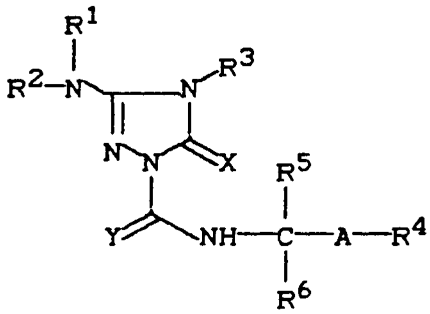

(I)

in welcher

A für einen Rest der Formel
$-CH_2-CH_2-$; $-CH_2-CH_2-CH_2-$;

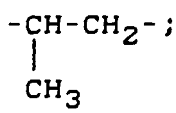
;
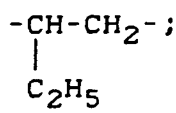
;
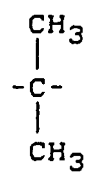

oder
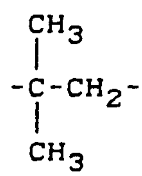

steht,

X für Sauerstoff oder Schwefel steht,

Y für Sauerstoff oder Schwefel steht,

$R^1$ für Wasserstoff oder Alkyl steht,

$R^2$ für Alkyl steht,

$R^3$ für Alkyl oder Alkenyl steht,

$R^4$ für jeweils gegebenenfalls substituiertes Cycloalkyl oder Aryl steht,

$R^5$ für Alkyl steht oder auch für Wasserstoff steht, für den Fall, daß nicht gleichzeitig A für $-CH_2-CH_2-$ steht und $R^4$ für unsubstituiertes Phenyl steht und

$R^6$ für Alkyl oder Cyano steht oder auch für Wasserstoff steht, für den Fall, daß A gleichzeitig für einen verzweigten Alkandiylrest der Formel

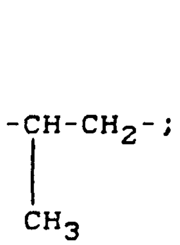
;
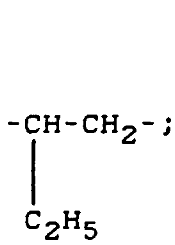
;
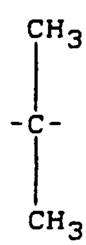
oder
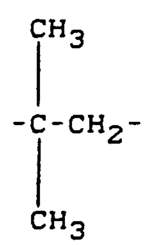

steht,
oder
$R^5$ und $R^6$ gemeinsam für einen zweifach verknüpften Alkandiylrest stehen.

**2.** Substituierte Triazolinone der allgemeinen Formel (I) gemäß Anspruch 1, dadurch gekennzeichnet, daß darin

A für einen Rest der Formel
$-CH_2-CH_2-$; $-CH_2-CH_2-CH_2-$;

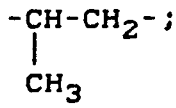
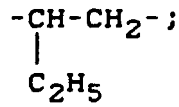
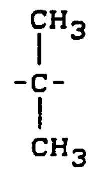

oder 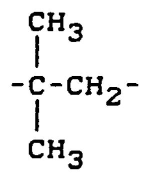

steht,

X für Sauerstoff oder Schwefel steht,

Y für Sauerstoff oder Schwefel steht,

$R^1$ für Wasserstoff oder für geradkettiges oder verzweigtes Alkyl mit 1 bis 6 Kohlenstoffatomen steht,

$R^2$ für geradkettiges oder verzweigtes Alkyl mit 1 bis 6 Kohlenstoffatomen steht,

$R^3$ für geradkettiges oder verzweigtes Alkyl mit 1 bis 6 Kohlenstoffatomen oder für geradkettiges oder verzweigtes Alkenyl mit 2 bis 6 Kohlenstoffatomen steht,

$R^4$ für gegebenenfalls einfach oder mehrfach, gleich oder verschieden substituiertes Cycloalkyl mit 3 bis 7 Kohlenstoffatomen steht, wobei als Substituenten infrage kommen: geradkettiges oder verzweigtes Alkyl mit 1 bis 4 Kohlenstoffatomen oder für jeweils gegebenenfalls einfach oder mehrfach, gleich oder verschieden substituiertes Phenyl oder Naphthyl steht, wobei als Substituenten infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kahlenstoffatomen, geradkettiges oder verzweigtes Alkoxycarbonyl oder Alkoximinoalkyl mit jeweils 1 bis 4 Kohlenstoffatomen in den einzelnen Alkylteilen; Dialkylamino mit jeweils 1 bis 4 Kohlenstoffatomen in den einzelnen geradkettigen oder verzweigten Alkylteilen, N-Alkanoylamino mit 1 bis 5 Kohlenstoffatomen im geradkettigen oder verzweigten Alkanoylteil, gegebenenfalls einfach oder mehrfach, gleich oder verschieden durch Halogen substituiertes, zweifach verknüpftes Dioxyalkylen mit 1 bis 3 Kohlenstoffatomen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl,

$R^5$ für geradkettiges oder verzweigtes Alkyl mit 1 bis 6 Kohlenstoffatomen steht oder auch für Wasserstoff steht, für den Fall, daß nicht gleichzeitig A für $-CH_2-CH_2-$ und $R^4$ für unsubstituiertes Phenyl steht und

$R^6$ für Cyano oder für geradkettiges oder verzweigtes alkyl mit 1 bis 6 Kohlenstoffatomen steht oder auch für Wasserstoff steht, für den Fall, daß A gleichzeitig für einen verzweigten Alkandiylrest der Formel

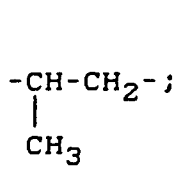 ; 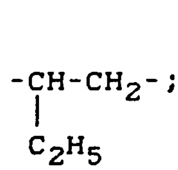 ; 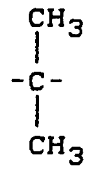 oder 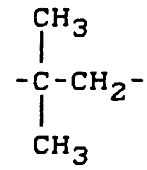

steht,
oder

$R^5$ und $R^6$ gemeinsam für einen zweifach verknüpften Alkandiylrest mit 2 bis 7 Kohlenstoffatomen stehen.

**3.** Substituierte Triazolinone der allgemeinen Formel (I) gemäß Anspruch 1, dadurch gekennzeichnet, daß darin

A für einen Rest der Formel
$-CH_2-CH_2-$; $-CH_2-CH_2-CH_2-$;

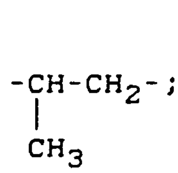 ; 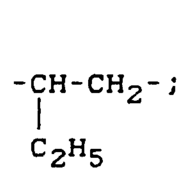 ; 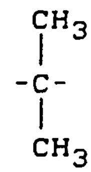

oder 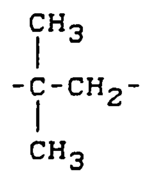

steht,

X für Sauerstoff oder Schwefel steht,

Y für Sauerstoff oder Schwefel steht,

$R^1$ für Wasserstoff oder für geradkettiges oder verzweigtes Alkyl mit 1 bis 4 Kohlenstoffatomen steht,

$R^2$ für geradkettiges oder verzweigtes Alkyl mit 1 bis 4 Kohlenstaffatomen steht,

$R^3$ für geradkettiges oder verzweigtes Alkyl mit 1 bis 4 Kohlenstoffatomen oder für geradkettiges oder verzweigtes Alkenyl mit 2 bis 5 Kohlenstoffatomen steht,

$R^4$ für gegebenenfalls ein- bis dreifach, gleich oder verschieden substituiertes Cyclohexyl steht, wobei als Substituenten infrage kommen: Methyl, Ethyl oder Isopropyl oder
für jeweils gegebenenfalls ein- bis dreifach, gleich oder verschieden substituiertes Phenyl oder Naphthyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethaxy, n- oder i-Propoxy, n-, i-, s- oder t-Butoxy, Methylthio, Ethylthio, Trifluormethyl, Difluormethyl, Trifluormethoxy, Trifluormethylthio, Methoxycarbonyl, Ethoxycarbonyl, Propoxycarbonyl, Dimethylamino, N-Acetamido, Methoximinomethyl, Methoximinoethyl, Ethoximinomethyl, Ethoximinoethyl, Dioxymethylen, Difluordioxymethylen, Tetrafluordioxyethylen oder gegebenenfalls ein- bis dreifach, gleich oder verschieden durch Fluor, Chlor, Brom, Methyl und/oder Ethyl substituiertes Phenyl,

$R^5$ für geradkettiges oder verzweigtes Alkyl mit 1 bis 4 Kohlenstoffatomen steht oder auch für Wasserstoff steht, für den Fall, daß nicht gleichzeitig A für
$-CH_2-CH_2-$ und $R^4$ für unsubstituiertes Phenyl steht und

$R^6$ für Cyano oder für geradkettiges oder verzweigtes Alkyl mit 1 bis 4 Kohlenstoffatomen steht oder auch für Wasserstoff steht, für den Fall, daß A gleichzeitig für einen verzweigten

Alkandiylrest der Formel

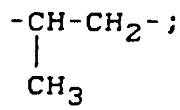 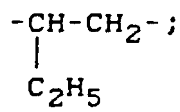 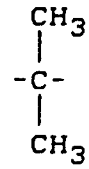 oder 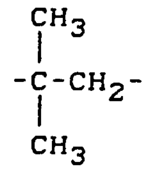

steht,

oder

$R^5$ und $R^6$ gemeinsam für einen zweifach verknüpften Alkandiylrest mit 4 bis 5 Kohlenstaffatomen stehen.

**4.** Substituierte Triazolinone der allgemeinen Formel (I) gemäß Anspruch 1, dadurch gekennzeichnet, daß darin

A für einen Rest der Formel $-CH_2-CH_2-$; $-CH_2-CH_2-CH_2-$;

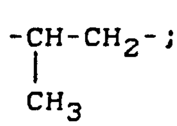 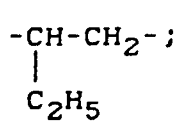 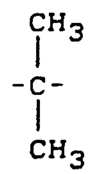

oder 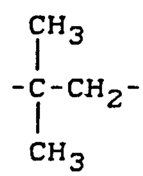

steht,

X für Sauerstoff steht,

Y für Sauerstoff steht,

$R^1$ für Wasserstoff oder für Methyl steht,

$R^2$ für Methyl steht,

$R^3$ für Methyl, Ethyl, n- oder i-Propyl, n-, i-, s-oder t-Butyl oder für Vinyl, Allyl, n- oder i-Butenyl steht,

$R^4$ für gegebenenfalls ein- bis dreifach durch Methyl und/oder Ethyl substituiertes Cyclohexyl steht, oder

für gegebenenfalls ein- bis dreifach, gleich oder verschieden substituiertes Phenyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Trifluormethyl, Difluormethyl, Trifluormethoxy, Trifluormethylthio, Methoxycarbonyl, Ethoxycarbonyl, Propoxycarbonyl, Dimethylamino, N-Acetamido, Dioxymethylen, Difluordioxymethylen, Tetrafluordioxyethylen oder gegebenenfalls ein- bis dreifach, gleich oder verschieden durch Fluor, Chlor, Brom, Methyl und/oder Ethyl substituiertes Phenyl,

$R^5$ für geradkettiges oder verzweigtes Alkyl mit 1 bis 4 Kohlenstoffatomen steht oder auch für Wasserstoff steht, für den Fall, daß nicht gleichzeitig A für $-CH_2-CH_2-$ und $R^4$ für unsubstituiertes Phenyl steht und

$R^6$ für Cyano oder für geradkettiges oder verzweigtes Alkyl mit 1 bis 4 Kohlenstoffatomen steht oder auch für Wasserstoff steht, für den Fall, daß A gleichzeitig für einen verzweigten Alkandiylrest der Formel

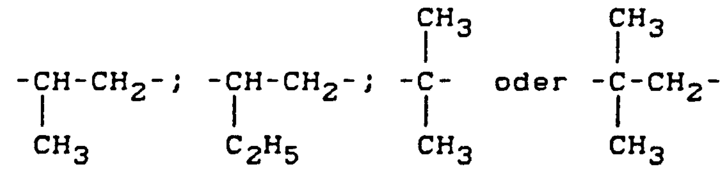

steht,
oder

$R^5$ und $R^6$ gemeinsam für einen zweifach verknüpften Pentandiylrest stehen.

**5.** Verfahren zur Herstellung von substituierten Triazolinonen der allgemeinen Formel (I),

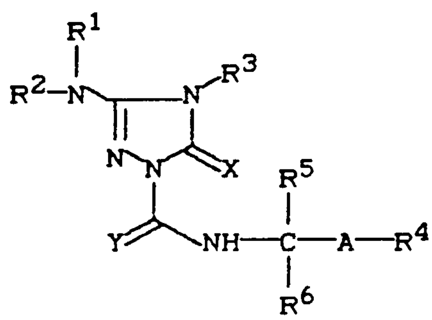

(I)

in welcher

A für einen Rest der Formel
$-CH_2-CH_2-$; $-CH_2-CH_2-CH_2-$;

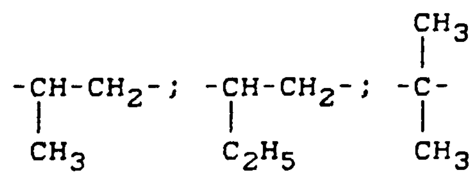

oder

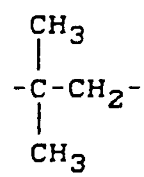

steht,

X für Sauerstoff oder Schwefel steht,

Y für Sauerstoff oder Schwefel steht,

$R^1$ für Wasserstoff oder Alkyl steht,

$R^2$ für Alkyl steht,

$R^3$ für Alkyl oder Alkenyl steht,

$R^4$ für jeweils gegebenenfalls substituiertes Cycloalkyl oder Aryl steht,

$R^5$ für Alkyl steht
oder auch für Wasserstoff steht, für den Fall, daß nicht gleichzeitig A für $-CH_2-CH_2-$ steht und $R^4$ für unsubstituiertes Phenyl steht und

$R^6$ für Alkyl oder Cyano steht oder auch für Wasserstoff steht, für den Fall, daß A gleichzeitig für einen verzweigten Alkandiylrest der Formel

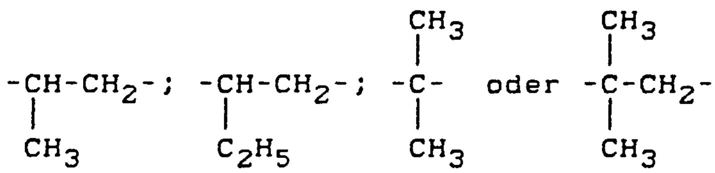

steht,
oder
$R^5$ und $R^6$ gemeinsam für einen zweifach verknüpften Alkandiylrest stehen,

dadurch gekennzeichnet, daß man

a) 1-Chlor-(thio)-carbonyltriazolinone der Formel (II),

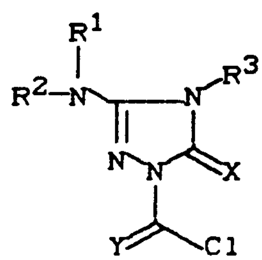

(II)

in welcher

$R^1$, $R^2$, $R^3$, X und Y die oben angegebene Bedeutung haben,

mit Aminen der Formel (III),

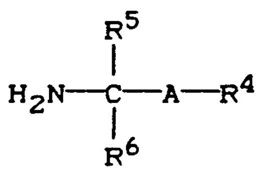

(III)

in welcher

$R^4$, $R^5$, $R^6$ und A die oben angegebene Bedeutung haben,

gegebenenfalls in Gegenwart eines Verdünnungsmittels und gegebenenfalls in Gegenwart eines Reaktionshilfsmittels umsetzt,

oder daß man

b) in 1-Stellung unsubstituierte Triazolinone der Formel (IV),

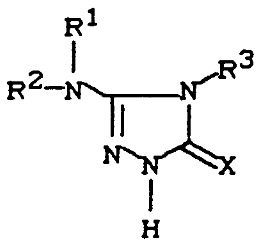

(IV)

in welcher

$R^1$, $R^2$, $R^3$ und X die oben angegebene Bedeutung haben,

mit Iso-(thio)-cyanaten der Formel (V),

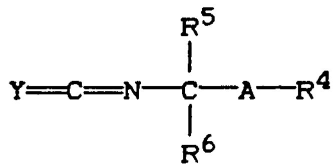

(V)

in welcher

A, Y, $R^4$, $R^5$ und $R^6$ die oben angegebene Bedeutung haben,

gegebenenfalls in Gegenwart eines Verdünnungsmittels und gegebenenfalls in Gegenwart eines Reaktionshilfsmittels umsetzt.

**6.** Herbizide Mittel, gekennzeichnet durch einen Gehalt an mindestens einem substituierten Triazolinon der allgemeinen Formel (I) gemäß Anspruch 1.

**7.** Verfahren zur Bekämpfung von Unkraut, dadurch gekennzeichnet, daß man ein substituiertes Triazolinon der Formel (I) gemäß Anspruch 1 auf die Unkräuter oder ihren Lebensraum einwirken läßt.

**8.** Verwendung von substituierten Triazolinonen der Formel (I) gemäß Anspruch 1 zur Bekämpfung von unerwünschtem Pflanzenwachstum.

**9.** Verfahren zur Herstellung von herbiziden Mitteln, dadurch gekennzeichnet, daß man substituierte Triazolinone der Formel (I) gemäß Anspruch 1 mit Streckmitteln und/oder oberflächenaktiven Miteln vermischt.

Europäisches Patentamt

# EUROPÄISCHER RECHERCHENBERICHT

Nummer der Anmeldung

EP 92 10 3589

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D<br><br>D | EP-A-0 283 876 (BAYER AG)<br>* Beispiele 94, 165, 197; Ansprüche 1-8 *<br>& DE-A-3 709 574<br>--- | 1-9 | C07D249/14<br>A01N47/38 |
| A | EP-A-0 398 096 (BAYER AG)<br>* Beispiele 67, 79; Ansprüche 1-8 *<br>--- | 1-9 | |
| P,A | EP-A-0 431 390 (BAYER AG)<br>* Beispiele 174, 177; Ansprüche 1-8 *<br>----- | 1-9 | |
| | | | **RECHERCHIERTE SACHGEBIETE (Int. Cl.5)** |
| | | | C07D<br>A01N |

Der vorliegende Recherchenbericht wurde für alle Patentansprüche erstellt

| Recherchenort | Abschlußdatum der Recherche | Prüfer |
|---|---|---|
| BERLIN | 21 MAI 1992 | van Amsterdam |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)